# EUROPÄISCHE PATENTSCHRIFT

(11) **EP 0 601 331 B1**
(45) Veröffentlichungstag und Bekanntmachung des Hinweises auf die Patenterteilung: **27.05.1998**
(21) Anmeldenummer: 93117689.5
(22) Anmeldetag: 02.11.1993
(51) Int. Cl.: A61N 5/06

(54) **Ganzkörper-Bräunungsgerät**
Device for tanning the whole body
Appareil de bronzage du corps entier

(30) Priorität: 05.12.1992 DE 9216593 U
(43) Veröffentlichungstag der Anmeldung: 15.06.1994
(73) Patentinhaber: JK-JOSEF KRATZ GmbH, D-53578 Windhagen (DE)
(72) Erfinder: Kratz, Walter, D-53783 Eitorf (DE)
(74) Vertreter: Heim, Hans-Karl, Dipl.-Ing.

(56) Entgegenhaltungen:
- EP-A- 0 292 410
- WO-A-87/00257
- DE-A- 2 821 891

## Beschreibung

Die Erfindung betrifft ein Ganzkörper-Bräunungsgerät gemäß dem Oberbegriff des Anspruchs 1. Ein solches Ganzkörper-Bräunungsgerät ist durch DE-A-2 821 891 bekannt.

Ein Ganzkörper-Bräunungsgerät besteht üblicherweise aus einem Unterteil, welches eine Liegefläche aufweist, und aus einem Oberteil, welches mit UV-Strahlern bestückt ist. Für eine Rundumbräunung kann auch das Unterteil mit UV-Strahlern versehen sein. Das Oberteil ist gewöhnlich höhenverstellbar, zumindest aber um eine Längsachse verschwenkbar, um einen bequemen Einstieg zu ermöglichen.

Das Oberteil ist im Abstand zum Unterteil an einer Deckenabhängung oder an einem Stativ befestigt. Es sind auch Ganzkörper-Bräunungsgeräte bekannt, bei denen das Ober- und Unterteil direkt verbunden sind, beispielsweise mit Hilfe von Scharnieren oder einer recht aufwendigen rückseitigen Konstruktion mit Schienenführung zur Höhenverstellbarkeit des Oberteils.

In einem zunehmenden Maße wurden die Ganzkörper-Bräunungsgeräte nicht nur technisch weiterentwickelt und mit Ausstattungen versehen, die den Bedienungskomfort und das Wohlbefinden erhöhen. Es wurde darüber hinaus auch die äußere Gestaltung in das Gesamtkonzept einbezogen und Ganzkörper-Bräunungsgeräte entwickelt, die hohen technischen und ästhetischen Ansprüchen gerecht werden.

Die für den privaten Bereich vorgesehenen und in Wohnlandschaften integrierbaren Ganzkörper-Bräunungsgeräte, aber auch die für eine gewerbliche Nutzung in Sonnenstudios, Kosmetikinstituten, Hotels und Saunas bestimmten Ganzkörper-Bräunungsgeräte sind nunmehr formschöne und repräsentative Geräte. Sie stellen jedoch gleichzeitig recht große, kompakte Einrichtungen dar, für die ein entsprechend großer Raum vorhanden sein muß. Dieser Raumbedarf wirkt sich auf die Mietkosten und somit auf die Betriebskosten eines gewerblichen Betreibers aus. Eine besonders aufwendige äußere Gestaltung ist außerdem mit höheren Material- und Lohnkosten verbunden, wodurch der Herstellungsaufwand insgesamt größer wird und der Preis für ein Ganzkörper-Bräunungsgerät ansteigt. Letztendlich ist die Verwendung von Kunstoffteilen, Spezialbeschichtungen und Farbanstrichen als eine Umweltbelastung nachteilig in Betracht zu ziehen.

Der Erfindung liegt die **Aufgabe** zugrunde, ein technisch hochwertiges Ganzkörper-Bräunungsgerät zu schaffen, welches besonders kostengünstig hergestellt und insbesondere bei gewerblicher Nutzung sehr kostensparend betrieben werden kann.

Erfindungsgemäß wird die Aufgabe durch die kennzeichnenden Merkmale des Anspruchs 1 gelöst. Zweckmäßige und vorteilhafte Ausgestaltungen und Weiterbildungen sind in den Unteransprüchen und in der Figurenbeschreibung enthalten.

Der Erfindung liegt der Grundgedanke zugrunde, Ganzkörper-Bräunungsgeräte unter Beibehaltung eines hohen technischen Standards und einer funktionsgerechten, ergonomischen Ausstattung von aufwendigen Elementen und Teilen, insbesondere von Gestaltungselementen des Gehäuses zu befreien und unter Berücksichtigung der räumlichen Gegebenheiten lediglich die sichtbaren, zugänglichen Bereiche, insbesondere in Frontbereichen mit einer Gehäuseverkleidung zu versehen.

Erfindungsgemäß weist ein Bräunungsgerät unverkleidete Oberflächenbereiche auf, welche frei von Verkleidungselementen gehalten sind. Die unverkleideten Bereiche sind in solchen Oberflächenbereichen vorgesehen, die beim bestimmungsgemäßen Gebrauch des Bräunungsgerätes nicht zugänglich sein müssen.

Erfindungsgemäß ist ein Ganzkörper-Bräunungsgerät als ein Ein- oder Anbaugerät konzipiert, das im Einbauzustand an mindestens eine externe Montagefläche angrenzt und das in diesem Oberflächenbereich frei von Verkleidungsteilen und Verkleidungselementen gehalten ist.

Als externe Montageflächen, an denen die unverkleideten Bereiche im eingebauten Zustand angrenzen, können normale Zimmerwände, aber auch Holzwände eines angrenzenden Saunaraumes oder beispielsweise in einem Sonnen- oder Kosmetikstudio auch Montagewände oder Raumteiler dienen.

Zweckmäßigerweise weist wenigstens das Unterteil unverkleidete Bereiche auf. Dabei wird davon ausgegangen, daß das Unterteil in der Regel in einem Raum an einem vorgesehenen Platz angeordnet ist und diesen Platz ohne kurzfristige Lageänderungen beibehält. In einer derartigen Anordnung liegt das Unterteil mit mindestens einer Seite, insbesondere mit der Rückseite oder mit mindestens einer Stirnseite an einer Wandfläche, im folgenden externe Montagefläche genannt, an. Fehlende bzw. nicht montierte Gehäuseteile können in diesen "offenen" Bereichen deshalb den ästhetischen Gesamteindruck eines Ganzkörper-Bräunungsgerätes nicht stören.

Vorzugsweise ist ein dreiseitiger Einbau eines erfindungsgemäßen Ganzkörper-Bräunungsgerätes vorgesehen, um eine besonders hohe Materialeinsparung infolge einer verkleidungsfreien Rückseite und verkleidungsfreier Stirnseiten zu erreichen. In diesem Fall können beide Stirnseiten und die Rückseite des Unterteils, die von Montageflächen begrenzt sind, an diesen Montageflächen auch lösbar, beispielsweise mit Schraubverbindungen, befestigt sein.

Es ist für eine Senkung des Herstellungsaufwandes sinnvoll, auch am Oberteil unverkleidete Bereiche in den von externen Montageflächen umgebenen Gehäuseseiten vorzusehen und insbesondere die Stirnseiten unverkleidet auszubilden.

In einer bevorzugten Weiterbildung sind das Ober- und Unterteil an den fluchtenden, unverkleideten Stirnseiten durch mindestens jeweils ein an den Stirnseiten anliegendes Verbindungselement miteinander verbunden, so daß eine aufwendige Hilfskonstruktion an der Rückseite oder auch eine Deckenabhängung nicht erforderlich ist.

Bevorzugt ist das Verbindungselement plattenförmig ausgebildet, welches auf einer Seite wesentliche Bereiche der unverkleideten Stirnseiten, insbesondere des Unterteils und gegebenenfalls auch eines Sockels unterhalb des Unterteils, bedeckt, und auf der anderen Seite an den externen Montagewänden liegt. Damit wird in einer zweckmäßigen Weise eine Halterung des Ganzkörper-Bräunungsgerätes an den externen Montagewänden erreicht, so daß eine stabile Einheit von Ganzkörper-Bräunungsgerät und Montagewänden gegeben ist.

In einer besonderen Gestaltungsvariante sind die Verbindungselemente als dreieckige Platten ausgebildet. Bei einem Verbindungselement in Form eines rechtwinkligen Dreiecks besteht die vorteilhafte Möglichkeit, die den rechten Winkel begrenzenden Seiten unmittelbar in dem von der rückseitigen Montagewand und der Bodenfläche gebildeten Winkel anzulegen und gegebenenfalls lösbar zu befestigen.

Es ist zweckmäßig, für das Oberteil eine höhenverstellbare Einrichtung und/oder einen Verschwenkmechanismus, beispielsweise Gasdruckfedern, im Bereich der Verbindungselemente anzuordnen. Zur Höhenverstellung kann auch eine Schienenführung vorgesehen sein.

Eine Frontseite ist als sichtbarer und zugänglicher Bereich des Ganzkörper-Bräunungsgerätes mit Verkleidungselementen oder Gehäuseflächen versehen. Im Bereich der Frontseite des Ganzkörper-Bräunungsgerätes ist ein Zugangsbereich vorzusehen.

Der Zugangsbereich, in dem eine Garderobe angeordnet sein kann, ist vorzugsweise innerhalb einer von Montagewänden gebildeten Bräunungskabine angeordnet.

Es ist vorteilhaft, auch einen Sockel des Ganzkörper-Bräunungsgerätes im Bereich der sichtbaren Frontseite mit Verkleidungselementen zu versehen.

Da prinzipiell das erfindungsgemäße Ganzkörper-Bräunungsgerät im eingebauten Zustand an nahezu ebene externe Wand- oder Montageflächen angrenzt, sind die unverkleideten, offenen Bereiche ebenfalls mit einer ebenen Außenkontur ohne überstehende Elemente auszubilden.

Zur lösbaren Befestigung an die Montageflächen oder -wände können Montageelemente, beispielweise Flansche mit Bohrungen für Steck- oder Schraubverbindungen, in den unverkleideten Bereichen vorgesehen sein. Diese Montageelemente können auch zur Halterung von Verkleidungsteilen oder Gehäuseelementen dienen, mit denen ein Ganzkörper-Bräunungsgerät, welches nachträglich frei im Raum aufgestellt werden soll, nachgerüstet werden muß. Möglich sind auch verstellbare, verschieb- oder schwenkbare Montageelemente, die ein Befestigen an Montagewände mit variablen Abstand ermöglichen.

Vorteilhaft ist eine unverkleidete Rückseite eines Einbau-Ganzkörper-Bräunungsgerätes auch in Bezug auf eine Belüftung oder Abluftabsaugung, weil ein Anschluß der entsprechenden Leitungen mit einem geringen Aufwand vorgenommen werden kann.

In Sonnenstudios und ähnlichen Einrichtungen, bei den insbesondere Ganzkörper-Bräunungsgeräte mit drei Seiten an externe Montagewände angestellt sind, kann eine effiziente zentrale Be- und Entlüftung mit optimierter Leitungsführung realisiert werden.

Die erfindungsgemäß ausgebildeten und an externe Montageflächen an- oder einbaubaren Ganzkörper-Bräunungsgeräte ermöglichen eine kostengünstige Herstellung und eine raumsparende Anordnung in Bräunungskabinen sowie eine raumsparende Anordnung mehrerer Bräunungskabinen in Sonnenstudios und dergleichen.

Die Erfindung wird nachstehend anhand einer Zeichnung weiter erläutert; in dieser zeigen in stark schematisierter Weise
- Fig. 1: eine frontseitige Ansicht von zwei erfindungsgemäßen Ganzkörper-Bräunungsgeräten, die in nebeneinander liegenden Bräunungskabinen angeordnet sind;
- Fig. 2: eine stirnseitige Ansicht von zwei, zu beiden Seiten einer Montagewand angeordneten erfindungsgemäßen Ganzkörper-Bräunungsgeräten;
- Fig. 3: eine Draufsicht auf zwei erfindungsgemäße Ganzkörper-Bräunungsgeräte gemäß Fig. 2 und
- Fig. 4: eine frontseitige Ansicht eines erfindungsgemäßen Ganzkörper-Bräunungsgerätes.

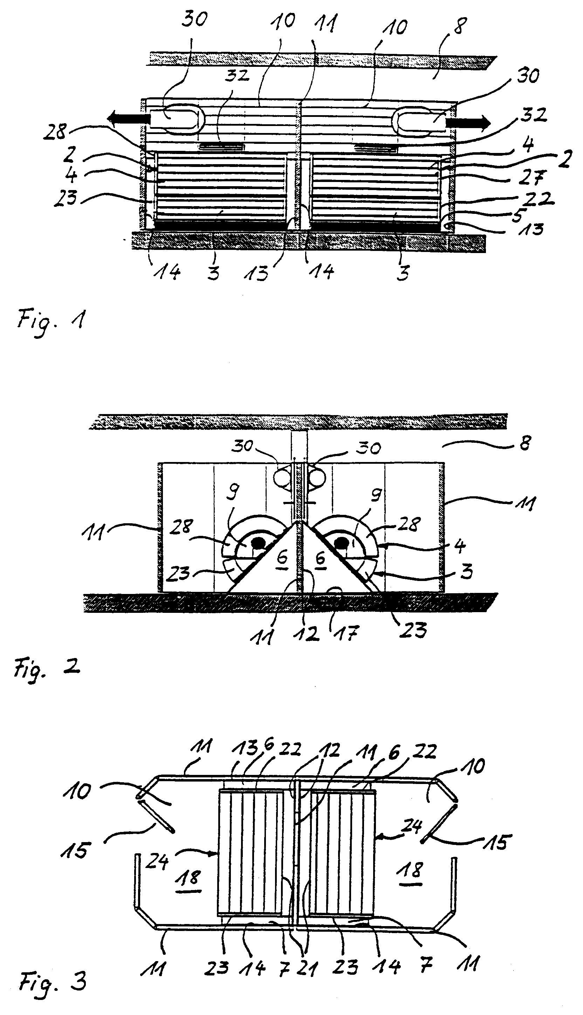

In den Figuren 1 bis 3 ist eine für ein Sonnenstudio geeignete Anordnung von Bräunungskabinen 10 mit jeweils einem erfindungsgemäß ausgebildeten Ganzkörper-Bräunungsgerät 2 dargestellt. In den ausschnittsweisen Darstellungen sind jeweils zwei Ganzkörper-Bräunungsgeräte 2 zu beiden Seiten einer externen Montagewand 11 angeordnet.

Die mit einem Unterteil 3 und einem Oberteil 4 versehenen Ganzkörper-Bräunungsgeräte 2 liegen mit Bereichen, die frei von Verkleidungsteilen gehalten sind, an externen Montageflächen 12, 13, 14 der Montagewände 11 an. Diese Bereiche sind die Rückseiten 21 und beide Stirnseiten 22, 23 des Unterteils 3 und auch die Stirnseiten 27, 28 des Oberteils 4 (Fig. 2 und 3). Das Unterteil 3 und das Oberteil 4 sind an den fluchtenden Stirnseiten 22, 27; 23, 28 durch zwei rechtwinklig ausgebildete, plattenförmige Verbindungselemente 6, 7 verbunden, die zur Halterung der eingebauten Ganzkörper-Bräunungsgeräte 2 dienen (Fig. 2). Das Oberteil 4 ist im Querschnitt nahezu halbkreisförmig und um eine Längsachse schwenkbar ausgebildet. Es bildet mit einem Unterteil 3 in einer Bräunungsposition, das heißt im abgeschwenkten Zustand, einen Bräunungstunnel 9, der von einer verkleideten Frontseite 24 aus zugänglich ist. Zu diesem Zweck ist jede von verstellbaren Montagewänden 11 gebildete Bräunungskabine 10 mit einem Zugangsbereich 18 versehen, der zwischen einer Tür 15 und dem Ganzkörper-Bräunungsgerät 2 liegt. Insbesondere aus der Fig. 1 und 3 geht die raumsparende Anordnung der eingebauten Ganzkörper-Bräungsgeräte 2 hervor.

Weitere Vorteile sind die Material- und Lohnkosteneinsparungen infolge der nicht herzustellenden und zu montierenden Verkleidungsteile. Vorteilhaft in technischer und in gestalterischer Hinsicht ist die Ausbildung und Anordnung der Verbindungselemente 6, 7, in die eine Einrichtung der Höhenverstellung oder eine Schwenkeinrichtung (nicht dargestellt) für das Oberteil 4 integriert sein kann. Die Verbindungselemente 6, 7 dienen in der dargestellten rechtwinkligen Ausbildung gleichzeitig als einpaßbare Halteelemente. Eine vorteilhafte kostengünstige Montage einer Be- und Entlüftung 30, 32 ist an der zwischen zwei Ganzkörper-Bräunungsgeräten 2 angeordneten Montagewand 11 möglich. Auch die Verbindung zu den in den Rückseiten 21 des Ganzkörper-Bräunungsgererätes 2 ausgebildeten Öffnungen (nicht dargestellt) für eine Frischluftzuführung oder auch Abluftabsaugung ist in einer einfachen und zeitsparenden Montage gewährleistet.

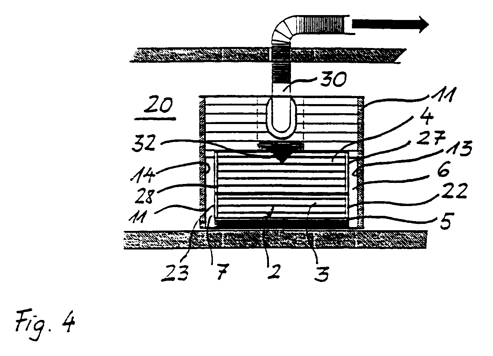

Fig. 4 zeigt ein im wesentlichen gleich ausgebildetes Ganzkörper-Bränungsgerät 2, welches beispielweise im privaten Bereich benutzt werden kann. Dieses Ganzkörper-Bräunungsgerät 2 ist ebenfalls an drei Seiten, nämlich an einer Rückseite 21 und an beiden Stirnseiten 22, 27; 23,28 des Unterteils 3 und des Oberteils 4 unverkleidet und liegt an externen Montagewänden 11 an. Ein Sockel 5 ist wie das Unterteil 3 und das Oberteil 4 im sichtbaren Frontbereich mit einer verkleideten Frontseite 24 versehen. Mit 32 ist eine Luftzufuhröffnung bezeichnet, die mit einer zentralen Belüftungseinrichtung verbunden ist.

## Patentansprüche

1. Ganzkörper-Bräunungsgerät,
mit
- einem Unterteil (3), welches als Liege ausgebildet ist,
- Seitenverkleidungselementen und
- einer Befestigungseinrichtung zum Befestigen des Unterteils (3) an einer externen Montagewand (12, 13, 14),
dadurch **gekennzeichnet**,
- daß eine Rückseite (21) und/oder zumindest eine Stirnseite (22, 23) des Unterteils (3) frei von Seitenverkleidungselementen gehalten und so ein offener Bereich gebildet ist,
- daß der offene Bereich mit einer ebenen Außenkontur ohne überstehende Elemente zur formschlüssigen Verbindung mit der Montagewand (12, 13, 14) ausgebildet ist, und
- daß die Befestigungseinrichtung an dem offenen Bereich des Unterteils (3) zum Befestigen des Unterteils (3) vorgesehen ist.

2. Ganzkörper-Bräunungsgerät nach Anspruch 1,
dadurch **gekennzeichnet**,
daß über dem Unterteil ein relative zum Unterteil (3) verstellbares Oberteil (4) angeordnet ist.

3. Ganzkörper-Bräunungsgerät nach Anspruch 2,
dadurch **gekennzeichnet,**
daß beide Stirnseiten (27, 28) des Oberteils (4) als unverkleidete Bereiche ausgebildet sind, daß die unverkleideten Stirnseiten (27, 28) des Oberteils (4) fluchtend zu den unverkleideten Stirnseiten (22, 23) des Unterteils (3) ausgebildet sind und daß mindestens jeweils ein Verbindungselement (6, 7) an den unverkleideten Stirnseiten (22, 27; 23, 28) anliegt und das Unter- und Oberteil (3, 4) verbindet.

4. Ganzkörper-Bräunungsgerät nach Anspruch 3,
dadurch **gekennzeichnet**,
daß das Verbindungselement (6, 7) plattenförmig ausgebildet ist und daß die unverkleideten Stirnseiten (22, 27; 23, 28) von dem plattenförmigen Verbindungselement (6, 7) wenigstens teilweise abgedeckt sind.

5. Ganzkörper-Bräunungsgerät nach Anspruch 3 oder 4,
dadurch **gekennzeichnet**,
daß das Verbindungselement (6, 7) als rechtwinkliges Dreieck ausgebildet ist, dessen den rechten Winkel begrenzenden Seiten bei einem eingebauten Ganzkörper-Bräunungsgerät (2) an einer rückseitigen Montagefläche (12) und an einer Bodenfläche (17) einer Bräunungskabine (10) zur Anlage zu bringen sind.

6. Ganzkörper-Bräunungsgerät nach einem der Ansprüche 3 bis 5,
dadurch **gekennzeichnet**,
daß die Verbindungselemente (6, 7) mit einer Einrichtung zum Höhenverstellen oder zum Aufschwenken des Oberteils (4) versehen sind.

7. Ganzkörper-Bräunungsgerät nach einem der vorhergehenden Ansprüche 2 bis 6,
dadurch **gekennzeichnet**,
daß von dem Unterteil (3) und dem in eine Bräunungsposition verstellten oder verschwenkten Oberteil (4) ein Bräunungstunnel (9) gebildet ist, daß ein Einstieg in den Bräunungstunnel (9) an verkleideten Frontseiten (24) des Unter- und Oberteils (3, 4) vorgesehen ist und daß ein Sockel (5) angeordnet ist, der lediglich in einem sichtbaren, vorderen Bereich mit Verkleidungselementen versehen ist.

8. Ganzkörper-Bräunungsgerät nach einem der vorhergehenden Ansprüche 2 bis 7,
dadurch **gekennzeichnet**,
daß die unverkleideten Bereiche (21, 22, 23; 27, 28) mit Montageelementen zur lösbaren Befestigung des Ganzkörper-Bräunungsgerätes (2) an externen Montageflächen (12, 13, 14) oder zur nachträglichen Befestigung von Verkleidungselementen versehen sind, daß als externe Montageflächen (12, 13, 14) für die unverkleideten Bereiche (21, 22, 23; 27, 28) eines eingebauten Ganzkörper-Bräunungsgerätes (2) verstellbare Montagewände (11) einer Bräunungskabine (10) vorgesehen sind und daß die Bräunungskabine (10) einen Zugangsbereich (18) aufweist, der vor den verkleideten Frontseiten (24) des Ganzkörper-Bräunungsgerätes (2) angeordnet ist.

9. Ganzkörper-Bräunungsgerät nach einem der vorhergehenden Ansprüche,
dadurch **gekennzeichnet**,
daß die unverkleideten Bereiche (21, 22, 23), insbesondere die Rückseite (21), mit mindestens einer Öffnung zur Zufuhr von Kühlluft, insbesondere einer zentralen Belüftungseinrichtung, versehen sind und/oder daß das Ganzkörper-Bräunungsgerät (2) mindestens eine Absaugöffnung aufweist, die mit einer zentralen Abluftleitung zu verbinden ist.

## Claims

1. Whole-body tanning device having a base (3), which is constructed as a bed, side panels and a fixing device for fixing the base (3) to an external assembly wall (12, 13, 14), characterized in that a back (21) and/or at least one end (22, 23) of the base (3) is kept free from side panels and consequently an open area is formed, that the open area is constructed with a planar outer contour without overhanging elements for positive connection to the assembly wall (12, 13, 14) and that the fixing device is provided at the open area of the base (3) for fixing said base (3).

2. Whole-body tanning device according to claim 1, characterized in that a top (4) adjustable relative to the base (3) is placed over said base.

3. Whole-body tanning device according to claim 2, characterized in that both ends (27, 28) of the top (4) are constructed as unpanelled areas, that the unpanelled ends (27, 28) of the top (4) are aligned with the unpanelled ends (22, 23) of the base (3) and that in each case at least one connecting element (6, 7) engages on the unpanelled ends (22, 27; 23, 28) and connects the base and top (3, 4).

4. Whole-body tanning device according to claim 3, characterized in that the connecting element (6, 7) has a plate-like construction and that the unpanelled ends (22, 27; 23, 28) are at least partly covered by the plate-like connecting element (6, 7).

5. Whole-body tanning device according to claim 3 or 4, characterized in that the connecting element (6, 7) is constructed as a right-angle triangle, whose sides defining the right angle in the case of an installed whole-body tanning device (2) are placed on a back assembly surface (12) and on a floor surface (17) of a tanning cabin (10).

6. Whole-body tanning device according to one of the claims 3 to 5, characterized in that the connecting elements (6, 7) are provided with a device for the vertical adjustment or swinging up of the top (4).

7. Whole-body tanning device according to one of the preceding claims 2 to 6, characterized in that a tanning tunnel (9) is formed by the base (3) and the top (4) adjusted or swung into a tanning position, that an entrance into the tanning tunnel (9) is provided on the panelled fronts (24) of the base and top (3, 4) and that a base member (5) is provided, which only has panels in a visible, front area.

8. Whole-body tanning device according to one of the preceding claims 2 to 7, characterized in that the unpanelled areas (21, 22, 23; 27, 28) are provided with assembly elements for the detachable fixing of the whole-body tanning device (2) to external assembly surfaces (12, 13, 14) or for the subsequent fixing of panels, that as external assembly surfaces (12, 13, 14) for the unpanelled areas (21, 22, 23; 27, 28) of an installed whole-body tanning device (12) are provided adjustable assembly walls (11) of a tanning cabin (10) and that the tanning cabin (10) has an access area (18), which is positioned in front of the panelled fronts (24) of the whole-body tanning device (2).

9. Whole-body tanning device according to one of the preceding claims, characterized in that the unpanelled areas (21, 22, 23), particularly the back, are provided with at least one opening for the supply of cooling air, particularly a central ventilation device and/or the whole-body tanning device (2) has at least one exhaust outlet for connection to a central used air line.

## Revendications

1. Appareil de bronzage intégral, comprenant :
- une partie inférieure (3) qui est conformée en couchette,
- des éléments latéraux d'habillage, et
- un dispositif de fixation pour fixer la partie inférieure (3) à une paroi de montage extérieure (12, 13, 14),
***caractérisé en ce qu***'une face arrière (21) et/ou au moins une face frontale (22, 23) de la partie inférieure (3) est maintenue libre d'éléments latéraux d'habillage et une zone ouverte est ainsi formée,
***en ce que*** la zone ouverte est conformée avec un contour extérieur plan sans élément en saillie, afin de réaliser un assemblage à engagement positif avec la paroi de montage (12, 13, 14), et
***en ce que*** le dispositif de fixation est prévu sur la zone ouverte de la partie inférieure (3) pour fixer la partie inférieure (3).

2. Appareil de bronzage intégral selon la Revendication 1, ***caractérisé en ce qu***'une partie supérieure (4) mobile par rapport à la partie inférieure (3) est placée au-dessus de la partie inférieure.

3. Appareil de bronzage intégral selon la Revendication 2, ***caractérisé en ce que*** les deux faces frontales (27, 28) de la partie supérieure (4) sont conformées en zones non habillées, ***en ce que*** les faces frontales non habillées (27, 28) de la partie supérieure (4) sont conformées alignées avec les faces frontales non habillées (22, 23) de la partie inférieure (3) et ***en ce qu'***au moins un élément de liaison (6, 7) s'appuie à chaque fois contre les faces frontales non habillées (22, 27 ; 23, 28) et relie les parties inférieure et supérieure (3, 4).

4. Appareil de bronzage intégral selon la Revendication 3, ***caractérisé en ce que*** l'élément de liaison (6, 7) est conformé en plaque et ***en ce que*** les faces frontales non habillées (22, 27 ; 23, 28) sont au moins partiellement recouvertes par l'élément de liaison (6, 7) en forme de plaque.

5. Appareil de bronzage intégral selon la Revendication 3 ou 4, ***caractérisé en ce que*** l'élément de liaison (6, 7) est conformé en triangle rectangle dont les côtés délimitant l'angle droit peuvent être amenés, dans un appareil de bronzage intégral installé, en appui contre une surface de montage arrière (12) et contre une surface de sol (17) d'une cabine de bronzage (10).

6. Appareil de bronzage intégral selon l'une quelconque des Revendications 3 à 5, ***caractérisé en ce que*** les éléments de liaison (6, 7) sont munis d'un dispositif de déplacement en hauteur ou de basculement de la partie supérieure (4).

7. Appareil de bronzage intégral selon l'une des Revendications 2 à 6, ***caractérisé en ce que*** la partie supérieure (4) déplacée ou basculée par rapport à la partie inférieure (3) et amenée dans une position de bronzage forme un tunnel de bronzage (9), ***en ce qu***'une entrée dans le tunnel de bronzage (9) est prévue sur des faces frontales habillées (24) des parties inférieure et supérieure (3, 4), et ***en ce qu***'un socle (5) est prévu, qui n'est muni d'éléments d'habillages que dans une zone avant visible.

8. Appareil de bronzage intégral selon l'une quelconque des Revendications 2 à 7, ***caractérisé en ce que*** les zones non habillées (21, 22, 23 ; 27, 28) sont munies d'éléments de montage pour une fixation amovible de l'appareil (2) de bronzage intégral sur des surfaces de montage externes (12, 13, 14) ou pour la fixation ultérieure d'éléments d'habillage, ***en ce que***, comme surfaces de montage externes (12, 13, 14) pour les zones non habillées (21, 22, 23 ; 27, 28) d'un appareil (2) de bronzage intégral installé, il est prévu des parois de montage réglables (11) d'une cabine de bronzage (10) et ***en ce que*** la cabine de bronzage (10) présente une zone d'accès (18) qui est placée devant les faces frontales habillées (24) de l'appareil (2) de bronzage intégral.

9. Appareil de bronzage intégral selon l'une quelconque des Revendications précédentes, ***caractérisé en ce que*** les zones non habillées (21, 22, 23), en particulier la face arrière (21), sont munies d'au moins une ouverture d'introduction d'air de refroidissement, en particulier d'un dispositif de ventilation central, et/ou ***en ce que*** l'appareil de bronzage intégral (2) présente au moins une ouverture d'aspiration, qui peut se relier à une conduite centrale d'évacuation d'air.
